# EUROPÄISCHE PATENTANMELDUNG

(11) **EP 2 008 644 A2**
(43) Veröffentlichungstag der Anmeldung: **31.12.2008**
(21) Anmeldenummer: 08104417.4
(22) Anmeldetag: 16.06.2008
(51) Int. Cl.: A61K 8/81, A61K 8/92, A61Q 1/10

(54) **Wimperntusche mit Styrol/Acrylat-Copolymeren**

(30) Priorität: 18.06.2007 DE 102007028498
(71) Anmelder: Beiersdorf AG, 20245 Hamburg (DE)
(72) Erfinder: Viala, Sophie, 50935, Köln (DE); Jaberi Motlagh, Sepideh, 22175, Hamburg (DE); Zoltowski, Craig, USA - Stamford, CT 06902 (US); Westphal, Tanja, 22307, Hamburg (DE)

(57) **Zusammenfassung**

Styrol/Acrylat-Copolymere mit einer Glasübergangstemperatur von kleiner oder gleich 20 °C in kosmetischen Zubereitungen.

## Beschreibung

Die vorliegende Erfindung betrifft Styrol/Acrylat-Copolymere mit einer Glasübergangstemperatur von kleiner oder gleich 20 °C in kosmetischen Zubereitungen.

Der Wunsch, schön und attraktiv auszusehen, ist seit Tausenden von Jahren in den Menschen verwurzelt. Auch wenn das Schönheitsideal im Laufe der Zeit Wandlungen erfahren hat, so ist das Streben nach einem makellosen Äußeren, immer das Ziel der Menschen gewesen, da ein sympathisches Erscheinungsbild ihr Selbstwertgefühl und die Anziehungskraft auf ihre Mitmenschen erhöht. Um dem Idealbild von Schönheit ein wenig näher zu kommen, verwenden die Menschen eine Reihe von Hilfsmitteln, insbesondere dekorative Kosmetika.

Der Begriff der dekorativen Kosmetik leitet vom lateinischen "decoratio" - das Hervorheben des Schönen - ab. Meist werden dabei mit Hilfe von Farbstoffen einzelne Körperpartien, insbesondere im Gesicht, hervorgehoben und farbliche Uneinheitlichkeiten abgemildert.

Das Augen-Make-up soll den Augen einen stärkeren Ausdruck verleihen. Das menschliche Auge wirkt insbesondere dann ausdrucksvoll, wenn der Farbkontrast zu den, das Auge umgebenen Wimpern besonders stark ist. Erreicht wird dieser Effekt durch das Färben der Wimpern und ihrer scheinbaren Verlängerung.

Zum Einfärben der Wimpern werden dem interessierten Verbrauchern heutzutage eine Vielzahl an unterschiedlichen Wimperntuschen (englisch: Mascara, abgeleitet aus dem arabischen mascha-ra = Possenreißer) hauptsächlich in den Farben schwarz, blau und braun angeboten. Weitere Inhaltsstoffe von Wimperntuschen sind die so genannten Haftstoffe, welche für einen festen Halt der Farbzubereitung auf der Wimper sorgen.

Damit Wimperntuschen nicht durch Schweiß oder Tränen aus den Wimpern während der Anwendung ausgewaschen werden und die, das Auge umgebende Haut einfärben, ist eine gewisse Feuchtigkeitsresistenz der Zubereitung erforderlich. Für besonders vorsichtige Anwender sind daher so genannte "wasserfeste" Wimperntuschen im Angebot. Die "Wasserfestigkeit" der Zubereitung wird dabei u.a. durch den Zusatz von polymeren Filmbildnern bewirkt.

Nachteilig am Stande der Technik ist jedoch der Umstand, dass die so genannten "wasserfesten" Wimperntuschen nur eine begrenzte Wasserfestigkeit aufweisen (siehe auch Vergleichsversuche).

Es war daher die Aufgabe der vorliegenden Erfindung eine Wimperntusche zu entwickeln, die eine erhöhte Wasser-, Schweiß- und Tränenfestigkeit aufweist.

Ein weiteres Problem des Standes der Technik von wasserfesten Wimperntuschen ist auch der Umstand, dass die Zubereitungen nur ungenügend auf der Wimper fixiert sind, relativ langsam trocknen und daher bei der Auftragung leicht verschmieren (englisch: smudge).

Es war daher die Aufgabe der vorliegenden Erfindung, eine Wimperntusche zu entwickeln, die weniger leicht verschmiert.

Durch den Zusatz von Filmbildnern zur Wimperntusche ändert sich regelmäßig die Stabilität und Konsistenz der Formulierung. Zubereitungen mit Filmbildner neigen dazu, insbesondere im trockenen Zustand körnig, brüchig und bröselig zu werden. Diese Effekte sind natürlich unerwünscht und sollten daher unterdrückt werden.

Es war daher die Aufgabe der vorliegenden Erfindung, eine Wimperntusche zu entwickeln, welche diese Effekte nicht mehr aufzeigt.

Überraschend gelöst werden die Aufgaben durch eine Wimperntusche-Zubereitung enthaltend Styrol/Acrylat-Copolymere mit einer Glasübergangstemperatur von kleiner oder gleich 20 °C.

Erfindungsgemäß ist darüber hinaus die Verwendung von Styrol/Acrylat-Copolymeren mit einer Glasübergangstemperatur von kleiner oder gleich 20°C zur Erhöhung der Wasserfestigkeit von kosmetischen Zubereitungen, insbesondere von Wimperntusche-Zubereitungen.

Zwar kennt der Fachmann die WO 01/54660, welche eine Zubereitung mit dem Styren/Acrylat-Copolymer "Joncryl^{®} 77" (welches eine Glasübergangstemperatur von Tg= 35°C aufweist) offenbart, doch konnte diese Schrift u.a. deshalb nicht den Weg zur vorliegenden Erfindung weisen, weil es sich nicht um eine Zubereitung handelt, die schmierig (und damit leicht verwischbar) sowie brüchig und damit kosmetisch vollkommen unattraktiv ist.

Ferner kennt der Fachmann die EP 1277456, EP 1101488, US 4423031 und EP 1249225, welche ebenfalls nicht den Weg zur vorliegenden Erfindung weisen konnten.

Die erfindungsgemäßen Zubereitungen sind einfach und preisgünstig herstellbar.

Die Glasübergangstemperatur wird erfindungsgemäß mit einem Mettler DSC 822e Gerät bestimmt. Die Probe wird dazu in einen 40µl fassenden Aluminiumtiegel eingefüllt und zweimal hintereinander im Temperaturbereich von -140°C bis +150°C bei einer Heizrate von 10°C/min vermessen. Zur Auswertung wird die zweite Aufheizkurve herangezogen.

Wie sich aus dem Vergleichsversuch (siehe unten) ergibt, ist die Wasserfestigkeit bei derartigen Copolymeren überraschend hoch.

Erfindungsgemäß vorteilhaft wird das erfindungsgemäße Copolymer aus den folgenden Monomeren aufgebaut:
(a) Styren und daraus abgeleitete Verbindungen
(b) Acrylsäure und/oder Methacrylsäure und/oder Verbindungen mit der Struktur H₂C=CR¹Y, wobei R¹ für Wasserstoff oder Methyl steht und Y sowohl -COOR²als auch COO-X⁺ bedeutet.
R² steht dabei für geradkettige oder verzweigte Alkylgruppen, geradkettige oder verzweigte Alkylengruppen, die jeweils auch substituiert sein können.
X⁺ bezeichnet Lithium-, natrium-, Kalium-, Magnesium-, Calcium-, Aluminium-, Ammonium-, Mono-/Di-/Tri/ und Tetraalkylammoniumionen.

Erfindungsgemäß bevorzugt sind Copolymere aus Acrylatmonomeren, Methacrylatmonomeren, Acrylsäure,Methacrylsäure und Styrol.

Die erfindungsgemäßen Styrol/Acrylat-Copolymere sind beispielsweise unter den Handelsnamen Joncryl 1532 und Joncryl ECO 2124 bei der Firma BASF Resins erhältlich.

Erfindungsgemäß besonders bevorzugt sind Copolymere aus Styrol, 2-ethylhexylacrylat, butylcrylat, methylmethacrylate und Acrylsäure (z.B. Joncryl ECO 2124) sowie Copolymere aus Sytrol, 2-ethylhexylacrylat, Isobutylcrylat, methylmethacrylate, butylacrylat, Acrylsäure, Methacrylsäure (z.B. Joncryl 1532).

Erfindungsgemäß vorteilhaft ist es, wenn die erfindungsgemäße Zubereitung Styrol/Acrylat-Copolymer in einer Konzentration von 0,1 bis 25 Gewichts-%, bezogen auf das Gesamtgewicht der Zubereitung, enthält.

Erfindungsgemäß bevorzugt ist es, wenn die erfindungsgemäße Zubereitung Styrol/Acrylat-Copolymer in einer Konzentration von 2 bis 15 Gewichts-%, bezogen auf das Gesamtgewicht der Zubereitung, enthält.

Erfindungsgemäß vorteilhafte Ausführungsformen der vorliegenden Erfindung sind dadurch gekennzeichnet, dass die Zubereitung ein oder mehrere Wachse mit einem Schmelzpunkt von 66 °C bis 88 °C aufweisen.

Es ist vorteilhaft im Sinne der vorliegenden Erfindung, wenn die Zubereitung ein oder mehrere Wachse enthalten, die einen Nadelpenetrationspunkt von 0,5 bis 6 dmm aufweisen.

Der Nadelpenetrationspunkt wird erfindungsgemäß mit der Methode ASTM D 1321 bei 25°C gemessen.

Es ist erfindungsgemäß vorteilhaft, wenn die erfindungsgemäße Zubereitung ein oder mehrere Wachse, welche einen Schmelzpunkt von 66 °C bis 88 °C aufweisen, in einer Konzentration von 5 bis 20 Gewichts-% , bezogen auf das Gesamtgewicht der Zubereitung, enthält.

Es ist erfindungsgemäß bevorzugt, wenn die erfindungsgemäße Zubereitung ein oder mehrere Wachse, welche einen Schmelzpunkt von 66 °C bis 88 °C aufweisen, in einer Konzentration von 8 bis 18 Gewichts-%, bezogen auf das Gesamtgewicht der Zubereitung, enthält.

Es ist erfindungsgemäß bevorzugt, wenn die Wachse mit einem Schmelzpunkt von 66 °C bis 88 °C, gewählt werden aus der Gruppe der Verbindungen Reiswachs, Sonnenblumenwachs, Candellilawachs, Carnaubawachs, Schellackwachs.

Es ist erfindungsgemäß vorteilhaft, wenn die Zubereitung ein oder mehrere Wachse, welche einen Nadelpenetrationspunkt von 0,5 bis 6 dmm aufweisen, in einer Konzentration von 5 bis 20 Gewichts-%, bezogen auf das Gesamtgewicht der Zubereitung, enthält.

Es ist erfindungsgemäß bevorzugt, wenn die Zubereitung ein oder mehrere Wachse, welche einen Nadelpenetrationspunkt von 0,5 bis 6 dmm aufweisen, in einer Konzentration von 8 bis 18 Gewichts-%, bezogen auf das Gesamtgewicht der Zubereitung, enthält.

Es ist erfindungsgemäß bevorzugt, wenn die Wachse mit einem Nadelpenetrationspunkt von 0,5 bis 6 dmm, gewählt werden aus der Gruppe der Verbindungen Reiswachs, Sonnenblumenwachs, Candellilawachs, Carnaubawachs, Schellackwachs.

Es ist erfindungsgemäß vorteilhaft, wenn die erfindungsgemäße Zubereitung Talkum in einer Konzentration von 0,5 bis 5 Gewichts-%, bezogen auf das Gesamtgewicht der Zubereitung, enthält.

Es ist erfindungsgemäß bevorzugt, wenn die erfindungsgemäße Zubereitung Talkum in einer Konzentration von 1 bis 3 Gewichts-%, bezogen auf das Gesamtgewicht der Zubereitung, enthält.

Es ist erfindungsgemäß bevorzugt, wenn die erfindungsgemäße Zubereitung Verdicker in einer Konzentration von 0,5 bis 5 Gewichts-%, bezogen auf das Gesamtgewicht der Zubereitung, enthält.

### Vergleichsversuche

Für den in-vitro Wasserfestigkeitstest wird weißes Aquarellpapier in Künstlerqualität (150 g/cm²) verwendet, auf das mit Hilfe einer Schablone eine 1cm x2cm große und 0,5 mm dicke Schicht aus der zu untersuchenden Wimperntusche aufgetragen wird.

Nachdem die Wimperntusche über Nacht bei Raumtemperatur getrocknet ist, wird 0,15g entmineralisiertes Wasser auf die getrocknete Wimperntusche getropft und für 10 Minuten einwirken gelassen.

Nach der Einwirkzeit wird ein zweites Stück Papier gleicher Qualität oben auf die Wimperntusche gelegt, und ein 2kg schweres Gewicht in Form einer Rolle gleichmäßig 10 mal über die beiden aufeinander liegenden Papiere gerollt.

Das obere Papier wird anschließend abgezogen, getrocknet und die Ausbreitung und Farbintensität der Abfärbung auf diesem ermittelt. Je mehr Farbe auf das obere Papier übertragen wurde, desto geringer ist die Wasserfestigkeit der Wimperntusche gewesen.

Die Auswertung erfolgt visuell anhand einer Vergleichsskala, kann aber auch über ein bildverarbeitendes Computerprogramm erfolgen.

### 1. Versuch

Es wurden 4 Wimperntusch-Zubereitungen hergestellt, die sich allein in der Art des eingesetzten Copolymers unterscheiden:

| **Rohstoff** | **Menge Var.1** | **Menge Var.2** | **Menge Var.3** | **Menge Var.4** |
|---|---|---|---|---|
| Magnesium Aluminium Silicate | 1,0 | 1,0 | 1,0 | 1,0 |
| Aqua | 33,4 | 54,9 | 44,9 | 44,9 |
| Styrene/Acrylate Copolymer + Aqua | 21,5 | - | - | - |
| (46,5 % Feststoffgehalt) | | | | |
| VP/Eicosene Copolymer | - | - | 10,0 | - |
| VP/Hexadecene Copolymer | - | - | - | 10,0 |
| Triethanolamine | 2,75 | 2,75 | 2,75 | 2,75 |
| Talc | 2,0 | 2,0 | 2,0 | 2,0 |
| Serica | 0,5 | 0,5 | 0,5 | 0,5 |
| CI 77499 | 10,0 | 10,0 | 10,0 | 10,0 |
| Butylene Glycol | 1,0 | 1,0 | 1,0 | 1,0 |
| Panthenol | 1,0 | 1,0 | 1,0 | 1,0 |
| BHT | 0,05 | 0,05 | 0,05 | 0,05 |
| Stearic Acid | 5,5 | 5,5 | 5,5 | 5,5 |
| Simethicone | 2,0 | 2,0 | 2,0 | 2,0 |
| Cyclomethicone | 2,0 | 2,0 | 2,0 | 2,0 |
| Phenoxyethanol+ Methylparaben+ Ethylparaben+ Butylparaben + Isopropyl-paraben +Propylparaben | 0,5 | 0,5 | 0,5 | 0,5 |
| Methylparaben | 0,3 | 0,3 | 0,3 | 0,3 |
| Cera carnauba | 4,0 | 4,0 | 4,0 | 4,0 |
| Helianthus Annuus Seed Wax | 9,0 | 9,0 | 9,0 | 9,0 |
| Shellac Cera | 3,5 | 3,5 | 3,5 | 3,5 |

Mit diesen Rezepturen wurde der oben beschriebene Wasserfestigkeitstest durchgeführt.

### Ergebnis: Zubereitung 1 hat die größte Wasserfestigkeit.

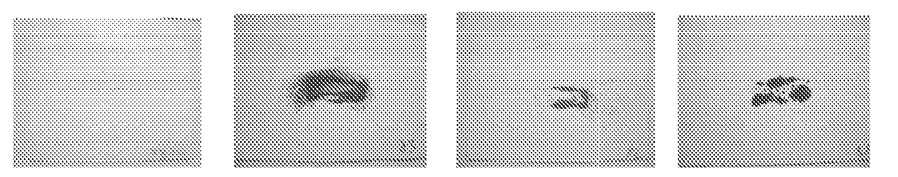

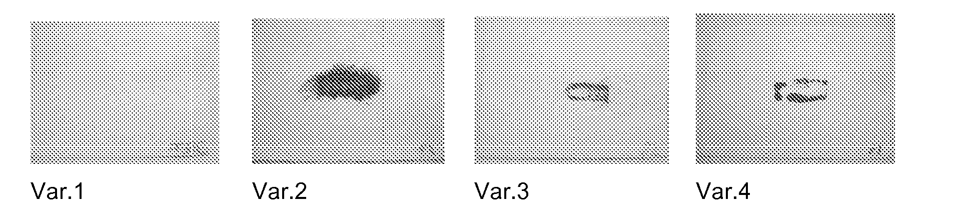

### 2. Versuch

Es wurden 3 Wimperntuschen mit gleichem Copolymer und Wachsen mit unterschiedlichem Schmelzpunkt/Nadelpenetrationspunkt hergestellt und die Wasserfestigkeit nach dem oben genannten Verfahren bestimmt:

| **Rohstoff** | **Menge Var.1** | **Menge Var.5** | **Menge Var.6** |
|---|---|---|---|
| Magnesium Aluminium Silicate | 1,0 | 1,0 | 1,0 |
| Aqua | 33,4 | 33,4 | 33,4 |
| Styrene/Acrylate Copolymer + Aqua | 21,5 | 21,5 | 21,5 |
| (46,5 % Feststoffgehalt) | | | |
| Triethanolamine | 2,75 | 2,75 | 2,75 |
| Talc | 2,0 | 2,0 | 2,0 |
| Serica | 0,5 | 0,5 | 0,5 |
| CI 77499 | 10,0 | 10,0 | 10,0 |
| Butylene Glycol | 1,0 | 1,0 | 1,0 |
| Panthenol | 1,0 | 1,0 | 1,0 |
| BHT | 0,05 | 0,05 | 0,05 |
| Stearic Acid | 5,5 | 5,5 | 5,5 |
| Simethicone | 2,0 | 2,0 | 2,0 |
| Cyclomethicone | 2,0 | 2,0 | 2,0 |
| Phenoxyethanol+ Methylparaben+ Ethylparaben+ Butylparaben + Isopropyl-paraben +Propylparaben | 0,5 | 0,5 | 0,5 |
| Methylparaben | 0,3 | 0,3 | 0,3 |
| Cera carnauba (80-86°C, 1,0-1,5 dmm) | 4,0 | 8,0 | 3,5 |
| Candelilla Cera (69-73°C, 2 dmm) | - | 4,5 | 9,0 |
| Helianthus Annuus Seed Wax (78-84°C, 2,5 dmm) | 9,0 | 4,0 | - |
| Oryza Sativa (78-87°C, 1dmm) | - | - | 4,0 |
| Shellac Cera (78-84°C, 4,0-5,0 dmm) | 3,5 | - | - |

### Ergebnis:

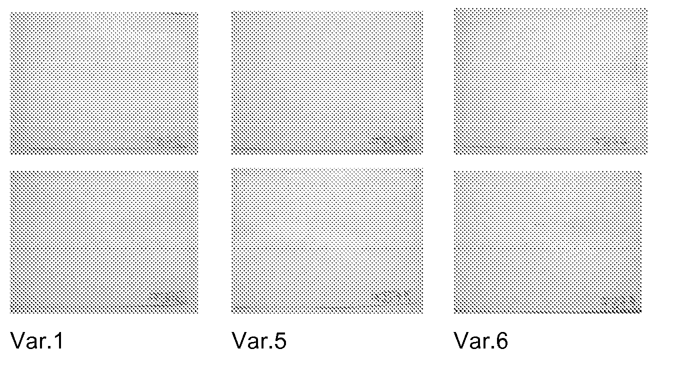

Die drei Formulierungen zeigen sehr gute Wasserfestigkeiten und sind hinsichtlich ihrer Wasserfestigkeit vergleichbar.

### Beispiele

Die nachfolgenden Beispiele sollen die vorliegende Erfindung verdeutlichen, ohne sie einzuschränken. Alle Mengenangaben, Anteile und Prozentanteile sind, soweit nicht anders angegeben, auf das Gewicht und die Gesamtmenge bzw. auf das Gesamtgewicht der Zubereitungen bezogen.

Die angegebene Menge bezieht sich bei Styrene/Acrylate Copolymer auf den Aktivgehalt des Polymers.

| | | | | | | | | | |
|---|---|---|---|---|---|---|---|---|---|
| Styrene/Acrylate Copolymer | 10 | 5 | 3 | 12 | 15 | 1 | 8 | 9 | 11 |
| Stearic acid | 5,5 | 5,5 | 5,5 | 5,5 | 5,5 | 5,5 | 2 | 2 | 2 |
| Polyglyceryl-3 Diisostearate | | | | | | | 0,75 | 1 | 0,7 5 |
| Cetyl Alcohol | 2 | | 2 | 1 | 2 | 1,5 | 1 | | |
| Lanolin Alcohol | | 2 | | 1 | | 0,5 | | | |
| Cera alba | 5 | | 4 | | 7 | | | | |
| Cera Carnauba | 1,25 | | | 1,5 | 3 | | 5 | | |
| Candelilla Cera | 5 | | 5 | 2,5 | | 2 | | 6 | 2 |
| Cera Microcristallina | 2 | | | | | 2 | 5 | | |
| Oryza sativa | | 3 | 2 | 1,25 | 4 | 2 | | 2 | 5 |
| Helianthus Annuus Seed Wax | | 5 | 1 | | 1 | 2 | 5 | | 2 |
| Shellac Cera | | 5 | 3 | | | | | 4 | 2 |
| Paraffin | | 1 | | 6 | | 2 | | | 2 |
| C18-38 Alkyl Hydroxystearoyl Stearate | | 1 | | 2 | | | | 3 | |
| Ozokerite | 2 | | | | | 5 | | | 2 |
| Cyclomethicone | | | 2 | | 2 | | | | |
| Simethicone | 2 | 2 | | 2 | | 2 | | | |
| Triethanolamine | 2,5 | 2,5 | 2,5 | 2,5 | 2,5 | 2,5 | | | |
| CI 28440 | 10 | 10 | 12 | 6,5 | 8 | 7,5 | 8 | 8,5 | 10 |
| Magnesium Aluminium Silicate | 2 | 2 | 2 | 2 | 2 | 2 | 2 | 2 | 2 |
| Talc | 2 | | | 1 | 0,5 | | 1 | | |
| Mica | | 2 | | | 0,5 | 2 | | | |
| Serica | | | 2 | 1 | 1 | | | | 1 |
| Active ingredients | 2 | 1,5 | 2 | 1 | 1 | 2,5 | 2 | 1,5 | 2 |
| Preservative systeme | 1 | 1 | 1 | 1 | 1 | 1 | 1 | 1 | 1 |
| Isododecan | | | | | | | 25 | 28 | 35 |
| Aqua | ad 100 | ad 100 | ad 100 | ad 100 | ad 100 | ad 100 | ad 100 | ad 100 | ad 10 0 |

## Patentansprüche

1. Wimperntusche-Zubereitung enthaltend Styrol/Acrylat-Copolymere mit einer Glasübergangstemperatur von kleiner oder gleich 20 °C.

2. Verwendung von Styrol/Acrylat-Copolymeren mit einer Glasübergangstemperatur von kleiner oder gleich 20°C zur Erhöhung der Wasserfestigkeit von kosmetischen Zubereitungen, insbesondere von Wimperntusche-Zubereitungen.

3. Zubereitung nach Anspruch 1 oder Verwendung nach Anspruch 2, **dadurch gekennzeichnet, dass** die Zubereitung ein oder mehrere Wachse mit einem Schmelzpunkt von 66 °C bis 88 °C aufweisen.

4. Zubereitung oder Verwendung nach einem der vorhergehenden Ansprüche, **dadurch gekennzeichnet, dass** die Zubereitung ein oder mehrere Wachse mit einem Nadelpenetrationspunkt von 0,5 bis 6 dmm aufweisen.

5. Zubereitung oder Verwendung nach einem der vorhergehenden Ansprüche, **dadurch gekennzeichnet, dass** die Zubereitung Styrol/Acrylat-Copolymer in einer Konzentration von0,1 bis 25 Gewichts-%, bezogen auf das Gesamtgewicht der Zubereitung, enthält.

6. Zubereitung oder Verwendung nach einem der vorhergehenden Ansprüche, **dadurch gekennzeichnet, dass** die Zubereitung ein oder mehrere Wachse, welche einen Schmelzpunkt von 66 °C bis 88 °C aufweisen, in einer Konzentration von 5 bis 20 Gewichts-%, bezogen auf das Gesamtgewicht der Zubereitung, enthalten.

7. Zubereitung oder Verwendung nach einem der vorhergehenden Ansprüche, **dadurch gekennzeichnet, dass** die Zubereitung ein oder mehrere Wachse, welche einen Nadelpenetrationspunkt von 0,5 bis 6 dmm aufweisen, in einer Konzentration von 5 bis 20 Gewichts-%, bezogen auf das Gesamtgewicht der Zubereitung, enthalten.

8. Zubereitung oder Verwendung nach einem der vorhergehenden Ansprüche, **dadurch gekennzeichnet, dass** die Wachse mit einem Schmelzpunkt von 66 °C bis 88 °C, gewählt werden aus der Gruppe der Verbindungen Reiswachs, Sonnenblumenwachs, Candellilawachs, Carnaubawachs, Schellackwachs.

9. Zubereitung oder Verwendung nach einem der vorhergehenden Ansprüche, **dadurch gekennzeichnet, dass** die Wachse mit einem Nadelpenetrationspunkt von 0,5 bis 6 dmm, gewählt werden aus der Gruppe der Verbindungen Reiswachs, Sonnenblumenwachs, Candellilawachs, Carnaubawachs, Schellackwachs.

10. Zubereitung oder Verwendung nach einem der vorhergehenden Ansprüche, **dadurch gekennzeichnet, dass** die Zubereitung Talkum in einer Konzentration von 0,5 bis 5 Gewichts-%, bezogen auf das Gesamtgewicht der Zubereitung, enthält.
